# EUROPÄISCHE PATENTSCHRIFT

(11) **EP 3 122 719 B1**
(45) Veröffentlichungstag und Bekanntmachung des Hinweises auf die Patenterteilung: **16.05.2018**
(21) Anmeldenummer: 15711536.1
(22) Anmeldetag: 24.03.2015
(51) Int. Cl.: C07C 263/10, C07C 265/14

(54) **VERFAHREN ZUM BETREIBEN EINER GASPHASENPHOSGENIERUNGSANLAGE**
METHOD FOR OPERATING A GAS PHASE PHOSGENATION SYSTEM
PROCÉDÉ DE FONCTIONNEMENT D'UNE INSTALLATION DE PHOSGÉNATION EN PHASE GAZEUSE

(30) Priorität: 27.03.2014 EP 14162004
(43) Veröffentlichungstag der Anmeldung: 01.02.2017
(73) Patentinhaber: Covestro Deutschland AG, 51373 Leverkusen (DE)
(72) Erfinder: KNAUF, Thomas, 41542 Dormagen (DE); LORENZ, Wolfgang, 41540 Dormagen (DE); STEFFENS, Friedhelm, 51373 Leverkusen (DE); BRUNS, Rainer, 51467 Bergisch Gladbach (DE); TAUBE, Wolfgang, 41470 Neuss (DE)
(74) Vertreter: Levpat
(86) Internationale Anmeldenummer: PCT/EP2015/056214
(87) Internationale Veröffentlichungsnummer: WO 2015/144681

(56) Entgegenhaltungen:
- WO-A1-2013/029918

## Beschreibung

Die Erfindung betrifft ein Verfahren zum Betreiben einer Gasphasenphosgenierungsanlage (**100**) zur Umsetzung eines Amins (**2**) mit Phosgen (**1**) zum korrespondierenden Isocyanat (**4**), bei dem zur Inbetriebnahme der Gasphasenphosgenierungsanlage (**100**) dieselbe zunächst mit Phosgen beschickt wird. Gleichzeitig mit oder nach der Erstbeschickung mit Phosgen werden die Aminzuführungseinrichtungen mit Hilfe eines heißen Inertgasstromes (**30**) inertisiert. Erst im Anschluss daran wird zum ersten Mal Amin zugegeben. Durch diese Maßnahmen sowie durch Einhalten eines Druckgefälles über die Amin- und Phosgenzuführungseinrichtungen zur Mischzone hin wird eine Rückvermischung von Phosgen in den Amin-haltigen Eduktstrom während der Inbetriebnahme verhindert.

Isocyanate werden in großen Mengen hergestellt und dienen hauptsächlich als Ausgangsstoffe zur Herstellung von Polyurethanen. Ihre Herstellung erfolgt zumeist durch Umsetzung der entsprechenden Amine mit Phosgen, wobei Phosgen im stöchiometrischen Überschuss eingesetzt wird. Die Umsetzung der Amine mit dem Phosgen kann sowohl in der Gasphase als auch in der Flüssigphase erfolgen. Die Verfahrensführung in der Gasphase, üblicherweise als Gasphasenphosgenierung bezeichnet, zeichnet sich dadurch aus, dass die Reaktionsbedingungen so gewählt werden, dass zumindest die Reaktionskomponenten Amin, Isocyanat und Phosgen, bevorzugt jedoch sämtliche Edukte, Produkte und Reaktionszwischenprodukte, bei den gewählten Bedingungen gasförmig sind. Vorteile der Gasphasenphosgenierung sind u. a. ein verringertes Phosgenaufkommen (sog. Phosgen-"Hold-Up"), die Vermeidung schwer phosgenierbarer Zwischenprodukte, erhöhte Reaktionsausbeuten und ein geringerer Energiebedarf, da mit weniger Lösungsmittel gearbeitet wird. Die vorliegende Erfindung betrifft ausschließlich die Gasphasenphosgenierung und betrifft insbesondere ein reibungsloses Verfahren zur Inbetriebnahme einer Gasphasenphosgenierungsanlage.

Es sind aus dem Stand der Technik verschiedene Verfahren zur Herstellung von Isocyanaten durch Umsetzung von Aminen mit Phosgen in der Gasphase bekannt. Wichtig für eine gute Verfahrensführung ist eine gute Vermischung der Edukte der Gasphasenphosgenierung. EP-A-0 289 840 beschreibt die Herstellung von Diisocyanaten durch Gasphasenphosgenierung, wobei die Herstellung erfindungsgemäß in einer turbulenten Strömung bei Temperaturen zwischen 200 °C und 600 °C in einem zylindrischen Raum ohne bewegte Teile stattfindet.

EP-A-0 570 799 betrifft ein Verfahren zur Herstellung von aromatischen Diisocyanaten, bei dem die Umsetzung des zugehörigen Diamins mit dem Phosgen in einem Rohrreaktor oberhalb der Siedetemperatur des Diamins innerhalb einer mittleren Kontaktzeit von 0,5 bis 5 Sekunden durchgeführt wird.

EP-A-0 699 657 beschreibt ein Verfahren zur Herstellung von aromatischen Diisocyanaten in der Gasphase, bei dem die Umsetzung des zugehörigen Diamins mit dem Phosgen in einem zwei Zonen enthaltenden Reaktor stattfindet, wobei die erste Zone, die etwa 20 % bis 80 % des Gesamtreaktorvolumens ausmacht, ideal vermischt ist und die zweite Zone, die 80 % bis 20 % des Gesamtreaktorvolumens ausmacht, durch eine Kolbenströmung charakterisiert werden kann. Bevorzugt wird die zweite Reaktionszone als Rohrreaktor ausgeführt.

Die Optimierung des Einsatzes von Rohrreaktoren für die Gasphasenphosgenierung, wie er grundlegend in der EP-A-0 570 799 unter Anwendung des Strahlmischerprinzips (Chemie-Ing.-Techn. 44 (1972) S. 1055, Abb. 10) offenbart wurde, ist Gegenstand zahlreicher Anmeldungen.

Nach der Lehre der EP-A-1 362 847 haben eine Vergleichmäßigung des über den Ringraum des Rohrreaktors zugeführten Eduktstromes und eine möglichst zentrale Zuführung beider Eduktströme in den Rohrreaktor einen großen positiven Einfluss auf die Stabilität der Reaktionszone und damit auf die Gasphasenreaktion insgesamt.

Wie in der EP-A-1 555 258 beschrieben, wird bei einer Vergrößerung der eingesetzten Rohrreaktoren auch eine Vergrößerung der Mischdüse, die häufig als Glattstrahldüse ausgebildet ist, notwendig. Mit der Vergrößerung des Durchmessers der Glattstrahldüse wird aber auch die Geschwindigkeit der Vermischung des Zentralstrahls durch den größeren erforderlichen Diffusionsweg verringert und die Gefahr von Rückvermischung erhöht, was wiederum zur Entstehung polymerer Verunreinigungen und damit zu festen Anbackungen im Reaktor führt. Nach der Lehre von EP-A-1 555 258 können die dargestellten Nachteile eliminiert werden, wenn der eine Eduktstrom über einen Ringspalt, der konzentrisch im Strom des anderen Eduktes angebracht ist, mit hoher Geschwindigkeit eingedüst wird. Dadurch werden der Diffusionsweg für die Vermischung klein und die Mischzeiten sehr kurz. Die Reaktion kann dann mit hoher Selektivität zum gewünschten Isocyanat ablaufen. Die Entstehung von polymeren Verunreinigungen und die Ausbildung von Anbackungen werden dadurch verringert.

Gemäß der Lehre von EP-A-1 526 129 hat eine Erhöhung der Turbulenz des Eduktstromes in der Zentraldüse einen positiven Einfluss auf die Durchmischung der Reaktanden und damit auf die Gasphasenreaktion insgesamt. Als Folge der besseren Durchmischung nimmt die Neigung zur Nebenproduktbildung ab.

EP-A-1 449 826 offenbart ein Verfahren zur Herstellung von Diisocyanaten durch Phosgenierung der entsprechenden Diamine, bei dem die dampfförmigen Diamine, gegebenenfalls verdünnt mit einem Inertgas oder mit den Dämpfen eines inerten Lösungsmittels, und Phosgen getrennt auf Temperaturen von 200 °C bis 600 °C erhitzt und in einem Rohrreaktor vermischt und zur Reaktion gebracht werden, wobei in dem Rohrreaktor eine Anzahl n2 von parallel zur Achse des Rohrreaktors ausgerichtete Düsen angeordnet sind, wobei der die Diamine enthaltende Strom dem Rohrreaktor über die n Düsen zugeführt wird und der Phosgenstrom dem Rohrreaktor über den verbleibenden freien Raum zugeführt wird.

Eine Weiterentwicklung des Einsatzes von Rohrreaktoren für die Gasphasenphosgenierung ist Gegenstand von WO 2007/028715. Der zum Einsatz kommende Reaktor weist eine Mischeinrichtung und einen Reaktionsraum auf. Gemäß der Lehre von WO 2007/028715 umfasst der Reaktionsraum im vorderen Bereich den Mischraum, in dem überwiegend die Vermischung der gasförmigen Edukte Phosgen und Amin, gegebenenfalls vermischt mit Inertmedium, stattfindet, was in der Regel begleitet ist vom Einsetzen der Reaktion. Gemäß der Lehre von WO 2007/028715 findet im hinteren Teil des Reaktionsraumes dann im Wesentlichen nur noch die Reaktion und höchstens untergeordnet die Vermischung statt. Bevorzugt werden in dem in WO 2007/028715 offenbarten Verfahren zur Strömungsrichtung rotationssymmetrische Reaktionsräume eingesetzt, die konstruktiv im Wesentlichen in bis zu vier Längenabschnitte entlang der Längsachse des Reaktors im Verlauf der Strömung zerlegt werden können, wobei sich die Längenabschnitte in der Größe der durchströmten Querschnittsfläche unterscheiden.

WO 2008/055898 offenbart ein Verfahren zur Herstellung von Isocyanaten durch Phosgenierung der entsprechenden Amine in der Gasphase in einem Reaktor, bei dem analog zu WO 2007/028715 der zum Einsatz kommende Reaktor eine Mischeinrichtung und einen Reaktionsraum aufweist, der rotationssymmetrische Reaktionsraum konstruktiv im Wesentlichen in bis zu vier Längenabschnitte entlang der Längsachse des Reaktors im Verlauf der Strömung zerlegt werden kann, wobei sich die Längenabschnitte in der Größe der durchströmten Querschnittsfläche unterscheiden. Gegenüber WO 2007/028715 werden die Veränderungen der durchströmten Querschnittsflächen jedoch nicht durch einen in einen Rohrreaktor installierten Volumenkörper, sondern durch eine entsprechende Erweiterung bzw. Einschnürung der Reaktoraußenwandung erreicht.

EP-A-1 275 639 offenbart als mögliche Verfahrensvariante zur Herstellung von Isocyanaten durch Phosgenierung der entsprechenden Amine mit Phosgen in der Gasphase ebenfalls den Einsatz eines Reaktors, bei dem der Reaktionsraum in Strömungsrichtung hinter der Vermischung der beiden Edukte eine Erweiterung der durchströmten Querschnittsfläche aufweist. Durch eine geeignet gewählte Erweiterung der Querschnittsfläche kann die Strömungsgeschwindigkeit der Reaktionsmischung über die Länge des Reaktors gerade konstant gehalten werden. Dadurch erhöht sich die zur Verfügung stehende Reaktionszeit bei gleich bleibender Länge des Reaktors.

EP-A-2 196 455 offenbart, dass Phosgen und die primären aromatischen Amine oberhalb der Siedetemperatur der Amine in einem Reaktor enthaltend einen zur Strömungsrichtung im Wesentlichen rotationssymmetrischen Reaktionsraum umgesetzt werden, wobei die querschnittsgemittelte Strömungsgeschwindigkeit des Reaktionsgemischs längs der Achse des im Wesentlichen rotationssymmetrischen Reaktionsraumes in dem Abschnitt des Reaktionsraums, in dem der Umsatz der Aminogruppen zu den Isocyanatgruppen zwischen 4 % und 80 % liegt, maximal 8 m/sec beträgt und wobei die querschnittsgemittelte Strömungsgeschwindigkeit des Reaktionsgemisches längs der Achse des im Wesentlichen rotationssymmetrischen Reaktionsraumes in diesem Abschnitt des Reaktionsraumes stets unterhalb der querschnittsgemittelten Strömungsgeschwindigkeit am Anfang dieses Abschnittes liegt.

In EP-A-1 935 876 ist ein Gasphasenverfahren zur Herstellung von Isocyanaten durch Umsetzung entsprechender primärer Amine mit Phosgen offenbart, in dem Phosgen und die primären Amine oberhalb der Siedetemperatur der Amine innerhalb einer mittleren Kontaktzeit von 0,05 bis 15 Sekunden umgesetzt werden, wobei die Umsetzung adiabat durchgeführt wird.

In EP-A-2 408 738 ist offenbart, wie man eine Phosgendissoziation in Chlor und Kohlenmonoxid infolge einer zu langen Verweilzeit der Phosgen-haltigen Ströme bei hoher Temperatur vermeiden kann. Durch Reduzierung der Verweilzeit des Phosgens bei Temperaturen grösser als 300 °C auf maximal 5 s und durch die Limitierung der Temperatur von mit Phosgen in Kontakt stehenden Wärmeüberträgerflächen von maximal 20 K oberhalb der einzustellenden Phosgentemperatur soll dies vermieden werden.

In EP-B-1 935 875 ist ein Verfahren zur Herstellung von Isocyanaten durch Umsetzung entsprechender primärer Amine mit Phosgen in der Gasphase offenbart, bei dem zum Abbruch der Reaktion das Reaktionsgemisch aus dem Reaktionsraum heraus durch eine Kühlstrecke geführt wird, in die hinein Flüssigkeiten eingedüst werden, wobei die direkte Kühlung in der Kühlstrecke einstufig in zwei oder mehreren hintereinander geschalteten Kühlzonen erfolgt (sog. "Quenchen" des Reaktionsgemisches).

WO 2013/029918 beschreibt ein Verfahren zur Herstellung von Isocyanaten durch Umsetzung der korrespondierenden Amine mit Phosgen, das auch bei unterschiedlichen Auslastungen der Gasphasenanlage ohne Probleme durchgeführt werden kann, insbesondere soll auch bei Fahren der Anlage im Teillastbereich das Mischen und/oder die Reaktion in dem jeweils optimierten Verweilzeitfenster erfolgen, indem das Verhältnis von Phosgen zu Amin erhöht wird oder ein oder mehrere inerte Stoffe dem Phosgen- und/oder Aminstrom zugesetzt werden. Das erfindungsgemäße Verfahren soll ermöglichen, eine bestehende Anlage bei unterschiedlichen Auslastungen mit gleichbleibender Produkt- und Verfahrensqualität zu betreiben. Dies soll die Anschaffung mehrerer Anlagen mit unterschiedlichen Nennkapazitäten einsparen.

Die Anmeldung lehrt, dass wesentliche Parameter einer Phosgenierung, wie insbesondere die Verweilzeiten der Reaktionspartner in den einzelnen Apparaten, für den Betrieb der Produktionsanlage bei Nennkapazität optimiert sind, was zu Problemen hinsichtlich Ausbeute und Produktreinheit führen kann, wenn die Anlage bei geringerer als Nennkapazität betrieben wird (vgl. S. 2, Z. 20 bis 36). Um die optimierten - engen - Verweilzeitfenster auch bei Teilauslastung (d. h. gegenüber Betrieb bei Nennkapazität verringertem *Amins*trom) erreichen zu können, wird vorgeschlagen, entweder den Phosgenstrom und/oder den Inertenanteil zu erhöhen (vgl. S. 3, Z. 5 bis 19), und zwar bevorzugt so, dass der Gesamtmengenstrom aller Komponenten im Wesentlichen dem bei Nennkapazität entspricht (vgl. S. 6, Z. 4 bis 8). Die Anmeldung erwähnt zwar Anfahrprozesse in der Beschreibung des Hintergrunds der beanspruchten Erfindung auf S. 2, offenbart jedoch keinerlei technische Lehre zum konkreten Handeln, wie denn eine *nicht in Betrieb* befindliche Produktionsanlage (d. h. Aminstrom und Phosgenstrom sind gleich *null*) am vorteilhaftesten auf den angestrebten Betriebszustand der Nennkapazität gebracht wird. Die in der Anmeldung offenbarten technischen Maßnahmen (d. h. die Erhöhung des Phosgenstroms und/oder des Inertenanteils) sind ausschließlich im Zusammenhang mit der Problematik *des Betriebs* (d. h. der Aminstrom ist signifikant *größer als* null) einer Produktionsanlage bei geringerer als Nennkapazität bzw. mit der Problematik, wie eine bei Nennkapazität *betriebene* Anlage vorteilhafterweise auf den Betrieb bei geringerer als Nennkapazität umgestellt werden kann (siehe die Beispiele), zu sehen.

Den beschriebenen Verfahren des Standes der Technik gelingt es zwar eine Phosgenierung durchzuführen, ohne dass es bei den Endprodukten zu einer Qualitätseinbuße kommt, jedoch werden von wenigen Ausnahmen abgesehen nur solche Verfahren beschrieben, die sich im Normalbetrieb befinden. Der Anfahrprozess bis zum Erreichen eines stabilen Betriebszustandes bei dem angestrebten Mengenstrom des umzusetzenden Amins, also die Inbetriebnahme einer Gasphasenphosgenierungsanlage, wird nicht beschrieben.

Dem Fachmann ist bekannt, dass ein kontinuierlich betriebener industrieller Prozess ausgehend von einer nicht in Betrieb befindlichen Produktionsanlage (z. B. nach einem wartungsbedingten Stillstand) nicht augenblicklich wieder auf die Prozessparameter vor dem Produktionsstillstand hochgefahren werden kann. Edukte und Apparaturen müssen aufgeheizt werden, Apparate müssen ggf. inertisiert werden, die Belastung der Apparate mit den Edukten wird allmählich auf den angestrebten Soll-Wert gesteigert. Die Inbetriebnahme (auch als Anfahren bezeichnet) einer Gasphasenphosgenierungsanlage ist ein im industriellen Alltag häufig auftretender Vorgang, der nicht zwangsläufig mit einem Öffnen oder einem anderen mechanischen Eingriff in die Phosgenieranlage verbunden sein muss. Das Anfahren zeichnet sich in der Praxis dadurch aus, dass es zu Abweichungen im Überschuss von Phosgen gegenüber Amin im Vergleich zum kontinuierlichen Betrieb bei Nennkapazität der Produktionsanlage kommen kann. Solche Abweichungen treten vor allen Dingen dann auf, wenn es z. B. durch Druckschwankungen zur Rückvermischung kommt. Dies ist insbesondere dann zu beobachten, wenn der aktuelle Mengenstrom an umzusetzendem Amin sehr klein ist im Vergleich zum angestrebten Mengenstrom an umzusetzendem Amin bei Nennkapazität der Anlage. Diese Mengenschwankungen im Verhältnis von Phosgen zu Amin sind nachteilig, da Feststoffe wie Polyharnstoff oder Aminhydrochloride ausfallen können. Darüber hinaus kann es bei unsachgemäßer Inbetriebnahme zur unerwünschten Bildung von Tröpfchen an Amin kommen. Die Inbetriebnahme einer Gasphasenphosgenierungsanlage ist daher ein kritischer Verfahrensschritt, da Fehler hierbei die eigentliche kontinuierliche Produktion empfindlich stören können (bspw. durch Erhöhung des Differenzdrucks, welcher nötig ist, um eine ausreichende Strömung der Edukte und Produkte durch die Anlage zu gewährleisten).

Es bestand daher trotz der vielfältigen Fortschritte auf dem Gebiet der Gasphasenphosgenierung Bedarf an weiteren Verbesserungen. Diesem Bedarf Rechnung tragend ist ein Gegenstand der vorliegenden Erfindung ein Verfahren zum Betreiben einer Gasphasenphosgenierungsanlage (**100**) zur Umsetzung eines Amins (**2**) mit Phosgen (**1**) zum korrespondierenden Isocyanat (**4**), wobei die Gasphasenphosgenierungsanlage (**100**) mindestens
(i) eine Vorrichtung **1000** zur Bereitstellung eines gasförmigen Phosgenstroms (**10**), optional umfassend neben Phosgen (**1**) einen Inertstoff (**3**),
(ii) eine Vorrichtung **2000** zur Bereitstellung eines gasförmigen Aminstroms (**20**), optional umfassend neben Amin (**2**) einen Inertstoff (**3**),
(iii) eine Mischzone (**3100**) zur Vermischung der Ströme **10** und **20,** wobei die Mischzone durch Einrichtungen (**1100, 2100**) jeweils mit der Vorrichtung **1000** und der Vorrichtung **2000** verbunden ist,
(iv) eine strömungstechnisch nach der Mischzone (**3100**) angeordnete Reaktionszone (**3200**) zur weiteren Umsetzung der zuvor vermischten Ströme **10** und **20,**
(v) eine strömungstechnisch nach der Reaktionszone (**3200**) angeordnete Reaktionsabbruchzone (**4000**) zur Beendigung der Umsetzung,
und optional
(vi) einen Aufarbeitungsteil (**5000**) umfassend Einrichtungen zur Rückgewinnung und Rückführung nicht umgesetzten Phosgens (**1"**) (**5100**) und Einrichtungen zur Gewinnung des hergestellten Isocyanats in Reinform (**5200**)
umfasst,
bei dem im Regelbetrieb der Gasphasenphosgenierungsanlage (**100**) das Phosgen (**1**) im Phosgengasstrom (**10**) bevorzugt eine Mischung aus Frisch-Phosgen (**1'**) und den Einrichtungen (**5100**) zurückgewonnenem Rezyklat-Phosgen (**1"**) ist,
wobei zur Inbetriebnahme der Gasphasenphosgenierungsanlage (**100**) die folgenden Schritte durchlaufen werden:
(I) Bereitstellung eines gasförmigen Phosgenstroms (**10**) einer Temperatur T₁₀ von 200 °C bis 600 °C, bevorzugt 200 °C bis 500 °C und besonders bevorzugt 250 °C bis 500 °C, mit einem absoluten Druck p₁ von 100 mbar bis 3.000 mbar, bevorzugt 150 mbar bis 2.800 mbar und besonders bevorzugt 200 mbar bis 2.500 mbar, in der Vorrichtung **1000** und kontinuierliches Einbringen dieses gasförmigen Phosgenstroms (**10**) durch die Einrichtung **1100** in die Mischzone (**3100**), die Reaktionszone (**3200**) und die Reaktionsabbruchzone (**4000**), wobei der Druck p₁₀ größer ist als der Druck p₃₁₀₀ in der Mischzone (**3100**);
(II) gleichzeitig mit oder nach Schritt (I),
   (a) Einleiten eines, bevorzugt bei Raumtemperatur und Normaldruck flüssig vorliegenden, Inertstoffs (**3**) einer Temperatur T₃ < 200 °C in die Vorrichtung **2000,** Erhitzen des Inertstoffs (**3**) in der Vorrichtung **2000** unter Erhalt eines Inertgasstroms (**30**), welcher durch die Einrichtung **2100,** die Mischzone (**3100**), die Reaktionszone (**3200**) und die Reaktionsabbruchzone (**4000**) geleitet wird oder
   (b) Einleiten eines Inertgasstroms (**30**)
      (b.1) in die Einrichtung **2100** und von dort durch die Mischzone (**3100**), die Reaktionszone (**3200**) und die Reaktionsabbruchzone (**4000**)
         oder bevorzugt
      (b.2) in die Vorrichtung **2000** und von dort durch die Einrichtung **2100,** die Mischzone (**3100**), die Reaktionszone (**3200**) und die Reaktionsabbruchzone (**4000**) wobei in Variante (a) und in Variante (b) der Inertgasstrom (**30**) eine Temperatur T₃₀ von 200 °C bis 600 °C, bevorzugt 200 °C bis 500 °C und besonders bevorzugt 250 °C bis 500 °C, und einen absoluten Druck p₃₀ von 100 mbar bis 3.000 mbar, bevorzugt 150 mbar bis 2.800 mbar und besonders bevorzugt 200 mbar bis 2.500 mbar, aufweist, wobei der Druck p₃₀ in beiden Fällen größer ist als der Druck p₃₁₀₀ in der Mischzone (**3100**);
(III) nach Schritt (II), Bereitstellung eines gasförmigen Aminstroms (**20**) einer Temperatur T₂₀ von 200 °C bis 600 °C, bevorzugt 200 °C bis 500 °C und besonders bevorzugt 250 °C bis 500 °C mit einem absoluten Druck p₂₀ von 100 mbar bis 3.000 mbar, bevorzugt 150 mbar bis 2.800 mbar und besonders bevorzugt 200 mbar bis 2.500 mbar, in der Vorrichtung **2000** und kontinuierliches Einbringen dieses gasförmigen Aminstroms (**20**) durch die Einrichtung **2100** in die Mischzone (**3100**), wobei der Druck p₂₀ größer ist als der Druck p₃₁₀₀ in der Mischzone (**3100**), und wobei die Zusammensetzung und der Massenfluss des Stroms (**20**) so auf die Zusammensetzung und den Massenfluss des Stroms (**10**) abgestimmt werden, dass in der Mischzone (**3100**) Phosgen (**1**) in einem stöchiometrischen Überschuss in Bezug auf die primären Aminogruppen des Amins (**2**) vorliegt.

Unter einer *Gasphasenphosgenierung* ist erfindungsgemäß eine Verfahrensführung zur Phosgenierung von Aminen zu den korrespondierenden Isocyanaten zu verstehen, bei der die Amine im gasförmigen Zustand zu den Isocyanaten reagieren und im Verlauf der Reaktion sämtliche vorhandenen Komponenten (Edukte, Produkte, Zwischenprodukte, ggf. Nebenprodukte, ggf. Inertstoffe) während des Durchgangs durch die Reaktionszone zu mindestens 95,0 Massen %, bevorzugt zu mindestens 98,0 Massen %, besonders bevorzugt zu mindestens 99,0 Massen %, ganz besonders bevorzugt zu mindestens 99,8 Massen % und speziell zu mindestens 99,9 Massen % jeweils bezogen auf die Gesamtmasse aller vorhandenen Komponenten, in der Gasphase verbleiben.

Geeignete *Amine (**2**)* sind dabei insbesondere Isophorondiamin, Hexamethylendiamin, Bis(p-aminocyclohexyl)methan, Toluylendiamin und Diphenylmethandiamin.

Im Rahmen der vorliegenden Erfindung umfasst der Ausdruck *"Inbetriebnahme einer Gasphasenphosgenierungsanlage"* alle Verfahrensschritte, die erforderlich sind, um eine außer Betrieb befindliche Gasphasenphosgenierungsanlage (bspw. nach einem wartungsbedingten Stillstand) auf die gewünschte Produktionskapazität, ausgedrückt als angestrebter Mengenstrom an umzusetzendem Amin, M'_{Soll}(**2**) [z. B. t(Amin)/h], zu bringen. Der Betrieb der Gasphasenproduktionsanlage (**100**) bei M'_{Soll}(**2**) wird im Rahmen dieser Erfindung als *Regelbetrieb* bezeichnet. M'_{Soll}(**2**) kann, muss aber nicht, dabei dem Wert von M'_{Soll}(**2**) bei Nennkapazität M'_{Nenn}(**2**) der Gasphasenproduktionsanlage (**100**) entsprechen. Die Nennkapazität einer Produktionsanlage wird in der Fachwelt in pro Jahr zu produzierender Tonnen an Produkt ("Jahrestonnen") angegeben, wobei alle planbaren Anlagenstillstände berücksichtigt werden.

Das Wort *"ein"* ist im Rahmen dieser Erfindung im Zusammenhang mit zählbaren Größen lediglich als unbestimmter Artikel und nur dann als Zahlwort zu verstehen, wenn dies ausdrücklich, etwa durch den Zusatz "*genau* ein", gesagt wird. Bspw. schließt der Ausdruck *"eine Reaktionszone"* die Möglichkeit des Vorhandenseins mehrerer (in Reihe oder parallel geschalteter) Reaktionszonen nicht aus.

Erfindungsgemäß wesentlich ist, dass zum Anfahren der Phosgenierungsanlage (**100**) dieselbe zunächst mit Phosgen beschickt wird. Beim Anfahren wird das Amin immer nach dem Phosgen zugegeben, wodurch ein Rückvermischen von Amin in den Phosgenweg verhindert und ein Überschuss an Phosgen gegenüber Amin sichergestellt wird. Vor und bevorzugt auch bei der Überführung des Amins in die Gasphase wird zusätzlich ein Inertstoff eingetragen, um beim Starten der Aminzufuhr ein Zurückströmen von Phosgen in die Aminzuführung (Rückvermischung) zu verhindern.

Nachstehend werden die Schritte der Erfindung detailliert erläutert. Verschiedene Ausführungsformen sind dabei beliebig miteinander kombinierbar, solange sich für den Fachmann aus dem Kontext nicht eindeutig das Gegenteil ergibt.

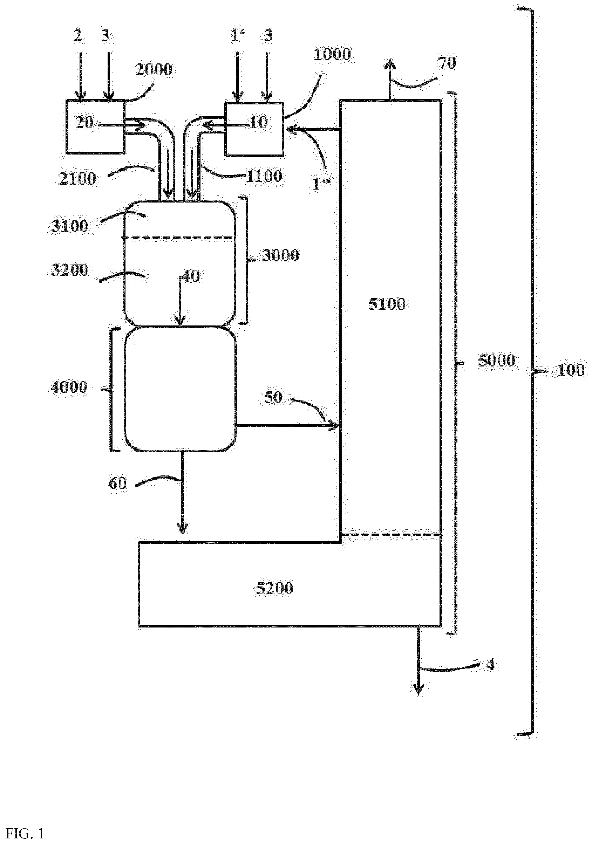

Erfindungsgemäß umfasst eine Gasphasenphosgenierungsanlage (**100**) mindestens die oben unter **(i) bis (v) aufgeführten Einrichtungen** (vgl. auch FIG. 1, welche die Einrichtungen einer erfindungsgemäß einzusetzenden Gasphasenphosgenierungsanlage (**100**) inklusive des bevorzugt vorhandenen Aufarbeitungsteils sowie die Stoffströme im Regelbetrieb zeigt).
(i) Als *Vorrichtung zur Bereitstellung eines gasförmigen Phosgenstroms (**1000**)* kann grundsätzlich jede aus dem Stand der Technik bekannte, für die Überführung von Phosgen in die Gasphase geeignete, Vorrichtung verwendet werden. Bevorzugt erfolgt die Phosgengaserzeugung durch Destillation oder partielle Verdampfung in einer Destillationskolonne, wie in DE 10 2009 032413 A1 in den Abschnitten [0081] bis [0118] beschrieben. Die Energiezufuhr im Sumpf der Kolonne kann durch jeden denkbaren Verdampfer erfolgen, wie zum Beispiel Naturumlaufverdampfer, Kletterverdampfer und Fallfilmverdampfer. Insbesondere bevorzugt sind Fallfilmverdampfer.
(ii) Als *Vorrichtung zur Bereitstellung eines gasförmigen Aminstroms (**2000**)* kann grundsätzlich jede aus dem Stand der Technik bekannte für die Überführung eines Amins in die Gasphase geeignete Vorrichtung, wie dem Fachmann bekannte Verdampfungsapparaturen, verwendet werden. In einer bevorzugten Ausführungsform umfasst die Vorrichtung **2000** eine Einrichtung zur Verdampfung und eine Einrichtung zur anschließenden Überhitzung des Amins (**2**). Ganz besonders bevorzugt sind mehrstufige Verdampfungs- und Überhitzungssysteme, in denen zwischen den Verdampfungs- und Überhitzungssystemen Tröpfchenabscheider eingebaut sind und/oder die Verdampfungsapparaturen auch die Funktion eines Tröpfchenabscheiders besitzen. Geeignete Tröpfchenabscheider sind z. B. beschrieben in "Droplet Separation", A. Bürkholz, VCH Verlagsgesellschaft, Weinheim - New York - Basel - Cambridge, 1989. Nach Verlassen des in Strömungsrichtung letzten Überhitzers wird der auf seine Solltemperatur vorgeheizte gasförmige Eduktstrom (**20**) dem Reaktionsraum zugeführt.
(iii) Der Aufbau einer erfindungsgemäß einsetzbaren *Mischzone (**3100**)* kann auf dem Fachmann bekannte Art und Weise erfolgen, bevorzugt wie in EP-A-2 196 455, insbesondere in den Absätzen [0047] bis [0049], und EP-A-1 935 876, insbesondere in den Absätzen [0027] bis [0029], beschrieben. Die Mischzone beginnt dort, wo im Regelbetrieb die Ströme (**10**) und (**20**) erstmals aufeinandertreffen.
(iv) Die in der Mischzone (**3100**) erstmals aufeinandertreffenden Amin- und Phosgengasströme werden in einem Verweilzeitapparat, der *Reaktionszone (**3200**),* weiter umgesetzt. Mischzone (**3100**) und Reaktionszone (**3200**) können bevorzugt auch in einem einzigen Apparat, dem *Reaktor (**3000**)* vereinigt sein wie in EP 2 196 455 A1, insbesondere in den Absätzen [0042] bis [0049], beschrieben.

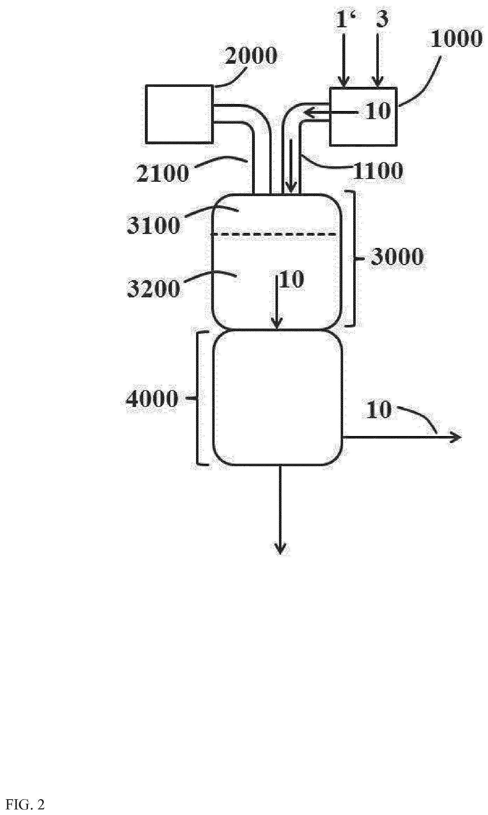

Die *Einrichtungen **1100** und **2100,*** welche die Vorrichtungen zur Bereitstellung des gasförmigen Phosgen- (**1000**) bzw. Amingasstroms (**2000**) mit der Mischzone (**3100**) verbinden, sind erfindungsgemäß solche Einrichtungen, welche sich zur Überführung des jeweiligen Gasstroms (**10**) bzw. (**20**) aus den Vorrichtungen **1000** bzw. **2000** in die Mischzone (**3100**) eignen. Diese Einrichtungen umfassen neben Rohrleitungen zum Transport der Gasströme bevorzugt auch Düsenvorrichtungen, die eine intensive Durchmischung von Phosgengasstrom (**10**) und Amingasstrom (**20**) in der Mischzone (**3100**) gewährleisten. Es ist möglich, jeden der Gasströme (**10**) und (**20**) einzeln in die Mischzone (**3100**) einzudüsen. Bevorzugt ist jedoch eine Ausführungsform, in welcher die Rohrleitungen der Einrichtungen **1100** und **2100** in eine gemeinsame Düsenvorrichtung einmünden (in FIG. 1 nicht gezeigt). In dieser Ausführungsform wird einer der beiden Gasströme, bevorzugt der Amingasstrom (**20**), der Mischzone (**3100**) über eine zentral in einem bevorzugt zylindrischen Behälter angeordnete Innendüse zugeführt. Der andere Gasstrom, bevorzugt der Phosgengasstrom (**10**), wird über den durch die Außenwand der Innendüse und der Innenwand des Behälters gebildeten Ringraum eingetragen. An der Austrittsöffnung der Innendüse (= Beginn der Mischzone) vermischen sich die beiden Gasströme. Eine solche Ausführungsform ist beispielsweise in FIG. 1 von EP-A-1 449 826 und in FIG. 1 von EP-A-1 362 847 dargestellt. In diesem Fall sind die Einrichtungen **1100** und **2100** teilweise in einander und in die Mischzone (**3100**) integriert. Es ist auch möglich, wie in FIG. 2 von EP-A-1 449 826 dargestellt, an Stelle einer einzigen Zentraldüse eine Anordnung aus mehreren Einzeldüsen zu verwenden. Weitere erfindungsgemäß einsetzbare Ausführungsformen für die Einrichtungen **1100** und **2100** sind beispielsweise in EP-A-2 196 455, insbesondere in den Absätzen [0047] bis [0048], und EP-A-1 935 876, insbesondere in den Absätzen [0027] und [0028], beschrieben.
(v) Erfindungsgemäß einsetzbare *Reaktionsabbruchzonen (**4000**)* sind dem Fachmann bekannt. Bevorzugt ist eine Ausführungsform, wie sie in EP 1 935 875 B1, insbesondere in den Absätzen [0024] und [0025], beschrieben ist. Bevorzugt wird die Reaktionsabbruchzone spätestens dann in Betrieb genommen, wenn in Schritt (III) erstmalig Strom **20** in die Mischzone (**3100**) eintritt. In der Reaktionsabbruchzone (**4000**) wird das Rohprodukt der Reaktion (**40**), welches neben dem Isocyanat (**4**) im Wesentlichen noch das Koppelprodukt Chlorwasserstoff und nicht umgesetztes Phosgen enthält, rasch abgekühlt, bevorzugt durch Eindüsen eines inerten Lösungsmittels (bevorzugt *ortho-*Dichlorbenzol, ODB), ggf. gemeinsam mit einem Teil des zuvor gebildeten und rezyklierten Isocyanats (**4**), in den Gasstrom (**40**). Bevorzugt wird das rohe Reaktionsprodukt (**40**) in der Reaktionsabbruchzone (**4000**) in einen gasförmigen Anteil (Brüden, **50**) und einen flüssigen Anteil (**60**) getrennt.

In einer besonders bevorzugten Ausgestaltung des erfindungsgemäßen Verfahrens wird das in der Reaktionsabbruchzone (**4000**) erhaltene Rohprodukt in derselben Gasphasenphosgenierungsanlage (**100**) aufgearbeitet, um das Isocyanat (**4**) aus dem flüssigen Gemisch (**60**) zu isolieren. In diesem Fall umfasst die Gasphasenphosgenierungsanlage (**100**) zusätzlich
(vi) einen *Aufarbeitungsteil (**5000**).*
   Geeignete Vorrichtungen zur Aufarbeitung sind in WO 2011/003532, insbesondere Seite 5, Zeile 19 bis Seite 28, Zeile 5, und in EP 1 371 636 B1, EP 1 371 635 B1 und EP 1 413 571 B1, jeweils das gesamte Dokument, beschrieben. Der Aufarbeitungsteil (**5000**) lässt sich unterteilen in *Einrichtungen zur Rückgewinnung und Rückführung nicht umgesetzten Phosgens (sowie zur Abtrennung des Koppelprodukts Chlorwasserstoff) (**5100**)* und in *Einrichtungen zur Gewinnung des hergestellten Isocyanats in Reinform (sowie ggf. zur Rückgewinnung inerten Lösungsmittels) (**5200**).* Der Aufarbeitungsteil ist in FIG. 1 nur schematisch angedeutet ohne die nachfolgend aufgeführten Details. Insbesondere bevorzugt umfasst der Aufarbeitungsteil (**5000**) eine *Auswaschkolonne (**5110**)* zur Entfernung von Isocyanat aus den Brüden (**50**) der Reaktionsabbruchsszone (**4000**) durch Waschen mit einem inertem Lösungsmittel, eine *Phosgenabsorptionskolonne (**5120**)* zur Wiedergewinnung von Phosgen aus den Brüden der Auswaschkolonne (**5110**) durch Absorption in einem inerten Lösungsmittel, wodurch Chlorwasserstoff und Inerte (**70**) vom Phosgen getrennt werden, eine *Phosgendesorptionskolonne (**5130**)* zur Trennung von Phosgen und inertem Lösungsmittel, eine *Lösungsmittelkolonne (**5210**)* insbesondere zur Abtrennung von Leichtsiedern (insbesondere inertem Lösungsmittel aus der Reaktionsabbruchzone) aus dem rohen Isocyanat, eine *Feinreinigungskolonne (**5220**)* insbesondere zur Abtrennung von Schwersiedern (z. B. Polyhamstoff-haltigen Rückständen) aus dem in der Lösungsmittelkolonne vorgereinigten Isocyanat, so dass gereinigtes Endprodukt erhalten wird.

Es ist möglich (in FIG. 1 nicht gezeigt), die Vorrichtung (**1000**) zur Bereitstellung eines gasförmigen Phosgenstroms (**10**) derart in die Phosgendesorptionskolonne (**5130**) zu integrieren, dass der aus der Aufarbeitung stammende lösungsmittelhaltige Phosgenstrom gemeinsam mit Frischphosgen (**1'**) verdampft und in der Phosgendesorptionskolonne (**5130**) destilliert wird. Das erhaltene gasförmige Phosgen wird über die Einrichtung **1100** der Mischzone zugeführt, während das abgetrennte inerte Lösungsmittel bevorzugt in die Auswaschkolonne (**5110**) und/oder die Phosgenabsorptionskolonne (**5120)** geführt wird.

In **Schritt (I)** des erfindungsgemäßen Verfahrens (siehe auch FIG. 2) wird in der Vorrichtung **1000** ein gasförmiger Phosgenstrom (**10**) bereitgestellt und durch die Einrichtung **1100** kontinuierlich in die Mischzone (**3100**), die Reaktionszone (**3200**) und die Reaktionsabbruchzone (**4000**) eingebracht, wobei der Druck p₁₀ größer ist als der Druck p₃₁₀₀ in der Mischzone (**3100**). Währenddessen ist die Zufuhr von Amingasstrom (**20**) unterbrochen. Bevorzugt sind während der Durchführung von Schritt (I) die Anlagenteile Einrichtung **1100,** Mischzone (**3100**), Reaktionszone (**3200**) und Reaktionsabbruchzone (**4000**) frei von Verunreinigungen, insbesondere frei von Lösungsmittel, das in vorangegangen Produktionszyklen möglicherweise aus dem Reaktionsabbruch durch rezyklierte Phosgenströme in der Phosgenierungsanlage verteilt oder als Inerstoff bewusst zugesetzt wurde. Bevorzugt wird der Phosgengas-Mengenstrom M'(**10**) bereits in Schritt (I) auf den späteren Soll-Wert M'_{Soll}(**10**) während der kontinuierlichen Produktion bei der angestrebten Kapazität eingestellt. Der gasförmige Phosgenstrom (**10**) kann neben Phosgen (**1**) noch einen Inertstoff (**3**) enthalten. In der Vorrichtung **1000** wird der Phosgengasstrom (**10**) auf eine Temperatur T₃₀ von 200 °C bis 600 °C bei einem absoluten Druck p₃₀ von 200 mbar bis 3.000 mbar erhitzt. Die Werte für T₁₀ und p₁₀ beziehen sich dabei auf den gesamten Strom (**10**), also ggf. auf die Mischung aus Phosgen (**1**) und Inertstoff (**3**), bei Austritt aus der Einrichtung **1100.** Erfindungsgemäß einsetzbare Inertstoffe (**3**) sind dabei neben solchen Stoffen, die bereits bei Raumtemperatur und Normaldruck gasförmig sind, wie Stickstoff, Helium oder Argon, auch die Dämpfe inerter organischer Lösungsmittel, die bei Raumtemperatur und Normaldruck flüssig sind, z. B. aromatische Kohlenwasserstoffe, ggf. mit Halogensubstitution, wie z. B. Chlorbenzol oder Dichlorbenzol (alle Isomere, bevorzugt *ortho-*Dichlorbenzol). Besonders bevorzugt wird Stickstoff zur Verdünnung des Phosgens eingesetzt. Der Anteil an Inertstoff (**3**) im Phosgengasstrom (**10**) kann dabei wie im Stand der Technik üblich gewählt werden. In Schritt (I) erfolgt das Aufheizen der Phosgenierungsanlage (**100**) mit dem Phosgenkreislauf, gefolgt vom Aufbau eines Inertgasstromes auf der Aminseite in Schritt (II).

In Schritt (I) stammt das Phosgen im Phosgenstrom (**10**) bevorzugt zumindest teilweise aus dem Aufarbeitungsteil der Gasphasenphosgenierungsanlage (**5000**) (Rezyklat-Phosgen (**1"**) aus dem vorhergehenden Produktionsszyklus). Die Rückgewinnung des Phosgens in der Aufarbeitung erfolgt dabei bevorzugt wie in WO 2011/003532, insbesondere Seite 5, Zeile 19 bis Seite 28, Zeile 5 beschrieben.

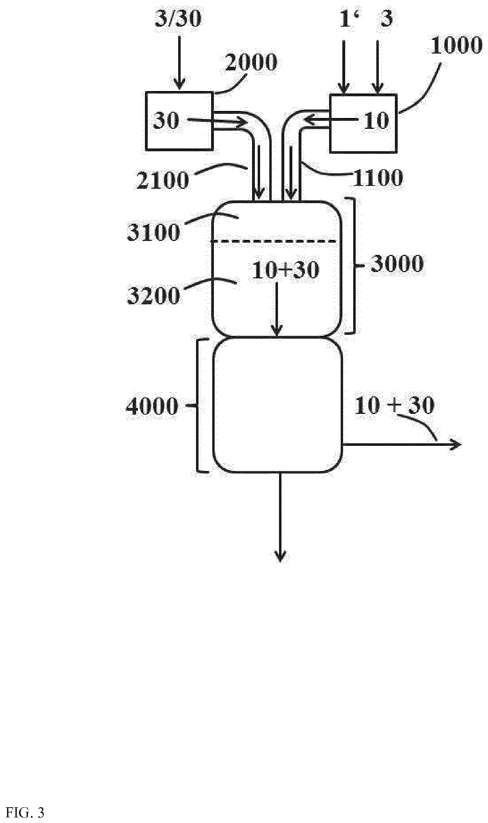

In **Schritt** (II) des erfindungsgemäßen Verfahrens (siehe auch FIG. 3) wird mindestens durch die Einrichtung **2100** (und von dort durch die Mischzone (**3100**), die Reaktionszone (**3200**) und die Reaktionsabbruchzone (**4000**)) ein Intertgasstrom (**30**) geführt. Bevorzugt werden die Vorrichtung **2000** *und* die Einrichtung **2100** dergestalt inertisiert, dass ein Inertgasstrom (**30**) aus der Vorrichtung **2000** in die Einrichtung **2100** (und von dort durch die Mischzone (**3100**), die Reaktionszone (**3200**) und die Reaktionsabbruchzone (**4000**)) geführt wird. Ggf. vorhandene Absperreinrichtungen zwischen der Vorrichtung **2000** und der Einrichtung **2100** (sowie zwischen der Einrichtung **2100** und der Mischzone **3100**) sind während dieses Schritts geöffnet, damit der Inertgasstrom (**30**) aus der Verdampfungsvorrichtung **2000** in die Einrichtung **2100** (und von dort in die Mischzone (**3100**)) strömen kann.

Die Inertisierung kann geschehen, indem (**a**) ein bei Raumtemperatur und Normaldruck bevorzugt flüssig vorliegender Inertstoff (**3**) einer Temperatur T₃ < 200 °C in die Vorrichtung **2000** eingeleitet und dort erhitzt wird. Diese Ausführungsform ist insbesondere dann vorteilhaft, wenn das Reaktionsgemisch während der Reaktion im Regelbetrieb mit den Dämpfen eines bei Raumtemperatur und Normaldruck flüssig vorliegenden Inertstoffs (**3**) verdünnt wird. Es ist möglich, den Inertstoff (**3**) flüssig in die Vorrichtung **2000** einzutragen und dort erst zu verdampfen. In der Variante (a) besonders geeignete Inertstoffe (**3**) sind inerte Lösungsmittel wie aromatische Kohlenwasserstoffe, ggf. mit Halogensubstitution, wie z. B. Chlorbenzol oder Dichlorbenzol (alle Isomere, bevorzugt *ortho*-Dichlorbenzol). In der Vorrichtung **2000** wird der Inertstoff (**3**) so erhitzt (d. h. bei Eintrag als Flüssigkeit verdampft), dass ein Inertgasstrom (**30**) erhalten wird, der eine Temperatur T₃₀ von 200 °C bis 600 °C, bevorzugt 200 °C bis 500 °C und besonders bevorzugt 250 °C bis 500 °C, und einen absoluten Druck p₃₀ von 100 mbar bis 3.000 mbar, bevorzugt 150 mbar bis 2.800 mbar und besonders bevorzugt 200 mbar bis 2.500 mbar, aufweist. Hierdurch werden die Vorrichtung **2000** und die nachgeschaltete Einrichtung **2100** aufgeheizt. Die Ströme (**3**) und (**30**) unterscheiden sich nicht in ihrer chemischen Zusammensetzung, sondern lediglich in Temperatur und ggf. Druck. Nachdem der in die Vorrichtung **2000** eingetragene Strom (**3**) erhitzt wurde, wird er als Strom (**30**) bezeichnet.

Es ist (**b**) auch möglich, den Inertgasstrom (**30**) außerhalb der Vorrichtung **2000** mit einer Temperatur T₃₀ von 200 °C bis 600 °C, bevorzugt 200 °C bis 500 °C und besonders bevorzugt 250 °C bis 500 °C, und einem absoluten Druck p₃₀ von 100 mbar bis 3.000 mbar, bevorzugt 150 mbar bis 2.800 mbar und besonders bevorzugt 200 mbar bis 2.500 mbar, bereitzustellen. Diese Ausführungsform ist insbesondere dann vorteilhaft, wenn das Reaktionsgemisch während der Reaktion im Regelbetrieb mit einem bei Raumtemperatur und Normaldruck bereits gasförmig vorliegenden Inertstoff wie Stickstoff, Helium oder Argon verdünnt wird. Der Inertgasstrom (**30**) kann (**b.1**) in die Einrichtung **2100** (und von dort durch die Mischzone (**3100**), die Reaktionszone (**3200**) und die Reaktionsabbruchzone (**4000**)) geführt werden. Die Einspeisung des Inertgasstroms (**30**) kann auch (**b.2**) in die Vorrichtung **2000** (und von dort durch Einrichtung **2100,** die Mischzone (**3100**), die Reaktionszone (**3200**) und die Reaktionsabbruchzone (**4000**)) erfolgen. Es ist in der Variante (**b.2**) auch möglich, den eingeleiteten Inertgasstrom (**30**) in der Vorrichtung **2000** im Rahmen der zuvor definierten Temperaturbereiche weiter aufzuheizen.

In einer bevorzugten Ausführungsform besteht der Inertgasstrom (**30**) aus dem gleichen Inertstoff (**3**), welcher ggf. zur Verdünnung des Phosgens (**1**) eingesetzt wird. Schritt (II) wird gleichzeitig mit oder nach Schritt (I) durchgeführt. Wird Schritt (II) nach Schritt (I) durchgeführt, so wird der in Schritt (I) eingestellte Phosgenstrom (**10**) während der Durchführung von Schritt (II) aufrechterhalten. Der optimale Mengenstrom an einzusetzendem Inertgasstrom (**30**) richtet sich nach der Größe der aufzuheizenden Anlagenteile und kann vom Fachmann leicht ermittelt werden. Durch Schritt (II) wird verhindert, dass beim Starten der Aminzufuhr in Schritt (III) die Vorrichtung **2100** Phosgen enthält und eine Kondensation des Amins (**2**) in der Mischzone (**3100**) oder in der Reaktionszone (**3200**) auftritt.

In **Schritt (III)** des erfindungsgemäßen Verfahrens wird in der Vorrichtung **2000** ein gasförmiger Aminstrom (**20**) bereitgestellt und durch die Einrichtung **2100** kontinuierlich in die Mischzone (**3100**) eingebracht. Bevorzugt wird hierbei ein Inertgasstrom (**30**) beibehalten. Vorteilhafterweise erfolgt dies, indem das Amin (**2**) mit einem Inertstoff (**3**), der entweder von vorneherein gasförmig (also als Inertgasstrom (**30**)) vorliegt oder in der Einrichtung **2000** zusammen mit dem Amin (**2**) in die Gasphase (also in einen Inertgasstrom (**30**)) überfuhrt wird, verdünnt wird. Der Amingasstrom (**20**) umfasst in dieser Ausführungsform also neben dem Amin (**2**) auch den Inertstoff (**3**). In der Vorrichtung **2000** wird der Amingasstrom (**20**) auf eine Temperatur T₂₀ von 200 °C bis 600 °C bei einem absoluten Druck p₂₀ von 100 mbar bis 3.000 mbar erhitzt. Die Werte für T₂₀ und p₂₀ beziehen sich dabei auf den gesamten Strom (**20**), also ggf. auf die Mischung aus Amin (**2**) und Inertstoff (**3**), bei Austritt aus der Einrichtung **2100,** d. h. in der bevorzugten Ausführungsform unter Einsatz einer Düse in der Einrichtung **2100,** an der Düsenmündung. Erfindungsgemäß einsetzbare Inertstoffe (**3**) sind dabei, wie im Fall des Phosgens, neben solchen Stoffen, die bereits bei Raumtemperatur und Normaldruck gasförmig sind, wie Stickstoff, Helium oder Argon, auch die Dämpfe inerter organischer Lösungsmittel, die bei Raumtemperatur und Normaldruck flüssig sind, z. B. aromatische Kohlenwasserstoffe, ggf. mit Halogensubstitution, wie z. B. Chlorbenzol oder Dichlorbenzol (alle Isomere, bevorzugt *ortho*-Dichlorbenzol). Besonders bevorzugt wird Stickstoff zur Verdünnung des Amins eingesetzt. Werden sowohl das Amin (**2**) als auch das Phosgen (**1**) mit einem Inertstoff (**3**) verdünnt, so ist es bevorzugt, dass als Inertgasstrom (**30**) der gleiche Inertstoff eingesetzt wird. Dieser ist bevorzugt Stickstoff. Der Anteil an Inertstoff (**3**) im Amingasstrom (**10**) kann dabei wie im Stand der Technik üblich gewählt werden. In Schritt (III) erfolgt das Verdampfen des Amins (**2**) in die permanent mit einem Inertgasstrom (**30**) gespülte Einrichtung **2100,** wobei die gesamte Phosgenierungsanlage von Eduktströmen bis Produktabnahme betriebsbereit sein muss. Zum Zeitpunkt des Austrittes des Amingasstroms (**20**) aus der Einrichtung **2100,** in der bevorzugten Ausführungsform unter Einsatz einer Düse aus der Mündung der Amindüse, in die Mischzone (**3100**) muss ein Phosgenüberschuss, bevorzugt von mindestens 150 %, gewährleistet sein. Die Temperatur in der Einrichtung **2100** und in der Mischzone (**3100**) sowie der Reaktionszone (**3200**) hat bevorzugt einen Wert oberhalb des Taupunktes des Amins (**2**). Der Druck in der Vorrichtung **2000** (Aminverdampfung) p₂₀ muss oberhalb des Drucks p₃₁₀₀ in der Mischzone (**3100**) liegen, welcher bevorzugt 80 mbar (absolut) bis 2.500 mbar (absolut) beträgt. Dies kann so geschehen, dass zunächst Amin (**2**) verdampft und dann durch (ggf. weitere) Zugabe eines Inertgasstroms (**30**) der Druck auf den gewünschten Wert p₂₀ eingestellt wird. Es ist bevorzugt, die Drücke p₁₀ und p₂₀ gleich zu wählen. Der so gewählte Druck muss größer sein als der in der Mischzone (**3100**) angestrebte Druck p₃₁₀₀, damit eine ausreichende Strömung der Gasströme (**10**) bzw. (**20**) in die Mischzone (**3100**) gewährleistet ist und eine Rückströmung in die jeweiligen Eduktdüsen sicher ausgeschlossen wird.

Erfindungsgemäß werden die Zusammensetzung und der Massenfluss des Stroms (**20**) so auf die Zusammensetzung und den Massenfluss des Stroms (**10**) abgestimmt, dass in der Mischzone (**3100**) Phosgen (**1**) in einem stöchiometrischen Überschuss in Bezug auf die primären Aminogruppen des Amins (**2**) vorliegt. Bevorzugt beträgt der auf die primären Aminogruppen des Amins bezogene Phosgenüberschuss 150 % bis 400 % der Theorie, besonders bevorzugt 160 % bis 350 % der Theorie und ganz besonders bevorzugt 170 % bis 300 % der Theorie. Theoretisch reagiert ein Mol Phosgen mit einem Mol Aminogruppen, d. h. theoretisch reagieren zwei Mol Phosgen mit einem Mol eines Diamins.

Der Massenfluss des Amingasstroms (**20**) wird stufenlos oder in Stufen, bevorzugt stufenlos, auf einen angestrebten Soll-Wert M'_{Soll}(**20**) gesteigert. Bevorzugt geschieht diese Steigerung so, dass innerhalb von weniger als 12 Stunden, bevorzugt weniger als 6 Stunden, besonders bevorzugt weniger als 3 Stunden und ganz besonders bevorzugt weniger als 1 Stunde mindestens 80 % des angestrebten Soll-Werts M'_{Soll}(**20**) erreicht werden. Hierdurch wird eine turbulente Rückvermischung von Phosgen, Amin oder Phosgen-/Amingemisch an der Stelle des Eintritts in die Mischzone (**3100**) vermieden.

Sollen zwei oder mehr Reaktionszonen (**3200**) parallel betrieben werden, ist es bevorzugt, diese nacheinander, wie vorstehend beschrieben, in Betrieb zu nehmen. Die Nebensysteme (wie die HCl-Absorption, Phosgenabsorption, ggf. Lösungsmittelaufarbeitung oder auch die Abgasbehandlung) müssen so dimensioniert sein, dass die anfallenden gasförmigen Koppel- und ggf. Nebenprodukte (insbesondere das Koppelprodukt Chlorwasserstoff) beim Anfahren unproblematisch aufgenommen und weiterverarbeitet werden können.

Sobald beide Mengenströme, M'(**10**) und M'(**20**), ihren jeweiligen Soll-Wert erreicht haben, kann die Gasphasenphosgenierungsanlage (**100**) nach einem aus dem Stand der Technik bekannten Verfahren weiterbetrieben werden. Bevorzugt wird hierzu das erzeugte Reaktionsgemisch unter Vermeidung von Rückvermischung kontinuierlich durch die Reaktionszone geleitet und darin bevorzugt bei einer Temperatur von 200 °C bis 600 °C und einem absoluten Druck von 80 mbar bis 2.500 mbar innerhalb einer mittleren Kontaktzeit von 0,05 bis 15 Sekunden adiabat oder isotherm, bevorzugt adiabat, zu einem gasförmigen Verfahrensprodukt enthaltend das gewünschte Isocyanat (**4**) umgesetzt. Geeignete Ausführungsformen sind in EP 2 196 455 B1 und EP 1 935 876 B1 beschrieben.

In der Reaktionsabbruchzone (**4000**) wird das aus der Reaktionszone (**3200**) austretende, gasförmige Verfahrensprodukt (**40**), rasch abgekühlt. Bevorzugt geschieht dies durch Kontaktieren mit einem inerten Lösungsmittel, dessen Temperatur unterhalb der Siedetemperatur des Isocyanats (**4**) und oberhalb der Zersetzungstemperatur des dem umgesetzten Amin entsprechenden Carbaminsäurechlorids gehalten wird. Geeignete Ausführungsformen sind in EP 1 935 875 B1 beschrieben. In diesem Schritt ggf. nicht kondensiertes Isocyanat (**4**) wird bevorzugt durch Waschen mit einer Waschflüssigkeit aus dem in der Reaktionsabbruchzone verbliebenen Gasgemisch abgetrennt und bevorzugt mit dem in der Reaktionsabbruchzone (**4000**) erhaltenen Kondensat (**60**) vereinigt. Eine geeignete Ausführungsform ist in EP 1 935 875 B1, insbesondere in den Absätzen [0024] und [0025], beschrieben.

Im Anschluss wird aus dem so erhaltenen rohen flüssigen Verfahrensprodukt das gewünschte Isocyanat (**4**) durch Destillation isoliert. Geeignete Ausführungsformen sind dem Fachmann bekannt und beispielsweise in WO 2013/139703, EP 1413 571 B1, EP 1 371 635 B1, EP 1 371 636 B1 beschrieben.

Fährt man alle Eduktwege gleichzeitig an, dann können die oben beschriebenen Probleme auftreten. Phosgen kann in die Einrichtung **2100** (bevorzugt Amindüse) strömen und es kann zu Blockierungen, Anbackungen mit Polyharnstoff etc. kommen. Zusätzlich wird der in der kontinuierlichen Produktion bei Nennkapazität angestrebte Phosgenüberschuss zumindest kurzzeitig signifikant unterschritten, wobei wiederum Nebenprodukte entstehen, weil die Strömungsgleichgewichte gestört sind und es zu unkontrollierter Vermischung kommt. Die Verweilzeit der Edukte im Reaktionsraum wird gestört, wenn beide Eduktströme gleichzeitig geöffnet werden.

Durch die erfindungsgemäßen Vorgehensweisen ergeben sich daher die folgenden Vorteile für das Anfahrprocedere einer Gasphasenphosgenierung:
i) Vermeidung von Verstopfungen in der Einrichtung **2100** (bevorzugt der Amindüse) und in der Mischzone (**3100**) und somit eine Vermeidung von sonst evtl. erforderlichem mehrfachem Anfahren, weil die Anlage zur Reinigung der Einrichtung **2100** (bevorzugt der Amindüse) abgefahren werden musste.
ii) Bedingt durch (i), Einsparung von Energie.
iii) Erhöhung der Produktivität der Anlage, weil nicht wegen der Entstehungen von Anbackungen und Ablagerungen mehrmals ab- und neu angefahren werden muss.
iv) Erhöhung der Anlagensicherheit, weil die thermische Belastung der Phosgenierungsanlage (**100**) durch die Verringerung von Anfahrvorgängen reduziert wird.
v) Verringerte Nebenproduktbildung sowie eine verkürzte thermische Belastung des Produktes und damit einhergehend eine Erhöhung der relativen Ausbeute.
vi) Vermeidung bzw. Verringerung von Niederschlägen, Anbackungen und Verstopfungen im Equipment (bspw. in der Vorrichtung **2000** und der Einrichtung **2100** oder in der Reaktionszone vor dem Reaktionsabbruch) und damit einhergehend eine Verlängerung der Standzeit des Verfahrens.
vii) Geringerer Abfall nach Reinigung des Equipments (bspw. weniger Polyharnstoff zu entfernen).
viii) Vermeidung von nicht spezifikationsgerechter Ware, die durch mehrfaches schlechtes An- und Abfahren entstehen kann. Solch eine qualitativ schlechte Anfahrware muss somit nicht mit qualitativ gutem Polyisocyanat verschnitten oder sogar im schlimmsten Fall verbrannt werden.

Somit ermöglicht das erfindungsgemäße Verfahren durch Vermeidung von Rückvermischungen von Phosgen (**1**) in den Amingasstrom (**20**) das Anfahren einer Gasphasenphosgenierungsanlage und die anschließende Aufarbeitung des entstandenen Roh-Isocyanats in technisch reibungsloser Weise mit verminderten oder im Idealfall ohne Ausfallzeiten mit direkt hoher Endproduktqualität.

### Beispiele

Gehaltsangaben in ppm oder % sind Massenanteile bezogen auf die Gesamtmasse des jeweiligen Stoff(strom)s.

### Allgemeine Bedingungen für die Herstellung von TDI bei "eingefahrener" Gasphasen-Produktionsanlage (100) (also nach erfolgter Inbetriebnahme)

### (Siehe auch FIG. 1 (vereinfachte Darstellung))

TDA (**2**) wird in einem Aminverdampfer (**2000**) gemeinsam mit Stickstoff (**3**) kontinuierlich verdampft. Der so erhaltene Amingasstrom (**20**), enthaltend 12 t/h gasförmiges TDA (**2**), wird über eine Leitung (**2100**), an deren zum Phosgenierungsreakor (**3000**) hin gelegenen Ende sich eine Amindüse befindet, kontinuierlich in den Phosgenierungsreaktor (**3000**) eingedüst. Die Verweilzeit des TDA-Stroms (**20**) von Verlassen des Verdampfers (**2000**) bis zum Austritt aus der Amindüse beträgt dabei 5 Sekunden. Gleichzeitig werden über einen Phosgengleichrichter, der, wie in EP-A-1 362 847 offenbart, eingesetzt wird, 61 t/h eines gasförmigen Phosgenstroms (**10**) kontinuierlich in den Phosgenierungsreaktor (**3000**) eingedüst. Das eingesetzte Phosgen ist eine Mischung aus frischem Phosgen (**1'**) und im Aufarbeitungsteil (**5000**) zurückgewonnenem Phosgen (1"). Dabei werden die beiden Edukte gut vermischt, und eine Rückvermischung unterbleibt. Die Temperatur des gasförmigen TDA-Stroms (**20**) am Düsenmund beträgt 380 °C (TDA hat ca. 1 Sekunde Verweilzeit bei dieser Temperatur im Zulauf zum Düsenmund). Das gasförmige Phosgen (**10**) hat eine Temperatur von 320 °C beim Verlassen des Phosgengleichrichters, wobei die Verweilzeit des heißen Phosgens zwischen letztem Phosgenüberhitzer und Phosgengleichrichter 2 Sekunden beträgt. Das gasförmige Gemisch aus den Strömen (**10**) und (**20**) hat eine Verweilzeit von 8 Sekunden im Gasphasenreaktor (**3000**) und reagiert bei einem absoluten Druck von 1.692 mbar zu gasförmigem Reaktionsgemisch (**40**). Die sich anschließende Reaktionsabbruchzone (**4000**) umfasst eine zweistufige "Quenche", in welcher das gasförmige Reaktionsgemisch (**40**) durch Einsprühen von *ortho*-Dichlorbenzol (ODB) auf 168 °C abgekühlt wird, sodass es kondensiert und sich im Sumpfbehälter (**4100**) ein Gemisch (**60**) aus Roh-TDI und ODB ansammelt. Überschüssiges Phosgen, bei der Reaktion entstandener Chlorwasserstoff und Inerte werden unter diesen Bedingungen aus dem Sumpfbehälter (**4100**) weitestgehend entgast, wobei der Mitriss an TDI mittels Einbauten vermindert wird. Dieser Prozessrestgasstrom (**50**) wird zur Wiedergewinnung von mitgerissenem TDI, Phosgen und Chlorwasserstoff, wie in WO 2011/003532, Seite 11, Zeile 24 bis 25 beschrieben, aufgearbeitet (**5100**). Das Gemisch (**60**) aus dem Sumpfbehälter (**4100**) wird, wie in EP 1 413 571 B1 beschrieben, aufgearbeitet (**5200**), wobei TDI (**4**) in einem Mengenstrom von 15,6 t/h erhalten wird.

So hergestelltes TDI (**4**) hat typischerweise eine Reinheit von > 99,97 % (Gaschromatographie, GC), einen Restlösemittelgehalt an ODB von < 5 ppm (GC), einen Restchlorgehalt von hydrolisierbarem Chlor von < 10 ppm (Titration gemäß ASTM D4663), eine Acidität von gebundenem Chlor < 5 ppm (Titration gemäß ASTM D5629), und die Farbzahl, gemessen als Hazenzahl, ist < 15 (bestimmt nach DIN EN ISO 6271).

### Vergleichsbeispiel 1: Inbetriebnahme einer Gasphasenphosgenierungsanlage (100) unter Einspeisung von Amin (2) vor Phosgen (1)

Eine Gasphasenphosgenierungsanlage (**100**) wird, wie oben beschrieben, betrieben. Nach einer Betriebsunterbrechung wird die Anlage wie folgt wieder in Betrieb genommen: Der Aminverdampfer (**2000**) und die Leitung (**2100**) einschließlich der Amindüse werden mit einem Stickstoffgasstrom (**30**) beaufschlagt, wobei eine Temperatur von 380 °C eingestellt wird. Der Phosgenierungsreaktor (**3000**) ist Edukt- und Produkt-frei und wird mit heißem Stickstoff (**30**) inertisiert. Die Aminverdampfung im Aminverdampfer (**2000**) wird gestartet, TDA wird bei 300 °C verdampft, in einem weiteren Wärmeaustauscher auf 410 °C erhitzt und als gasförmiges TDA (**20**) bei einem absoluten Druck von 1.683 mbar durch die Amindüse in den Phosgenierungsreaktor (**3000**) eingedüst. Die Menge an TDA (**2**), die während einer geplanten Anfahrzeit von 45 Minuten in den Phosgenierungsreaktor (**3000**) eingeleitet wird, soll stufenlos von 0 t/h auf 12 t/h erhöht werden. 5 Minuten nach Beginn der Aminzufuhr wird ein Phosgengasstrom (**10**) mit einem Massenfluss von 61 t/h, einer Temperatur von 320 °C und einem absoluten Druck von 1.683 mbar am Reaktoreintritt in den Phosgenierungsreaktor (**3000**) eingedüst. Nach 20 Minuten muss die Anlage abgestellt werden, weil der Differenzdruck zwischen Eintritt der Edukte TDA-Gasstrom (**20**) und Phosgengasstrom (**10**) in den Phosgenierungsreaktor (**3000**) und Brüdengasaustritt des Sumpfes (**4100**) rasant auf über 1.000 mbar, statt 10 mbar bei Normalbetrieb, angestiegen ist, und die benötigte Verdampfungsenergie zum Verdampfen von Phosgen (**1**) und TDA (**2**) nicht mehr aufgebracht werden kann. Die Inbetriebnahme wird durch Abschalten der Phosgen- und TDA-Zufuhr unterbrochen. Nach dem Öffnen der Phosgenierungsanlage werden ein mit TDA und Polyharnstoffen verstopfter Demister im Sumpf (**4100**) und mit großflächigen Ablagerungen belegte Oberflächen an den Quenchen (**4000**) gefunden.

### Vergleichsbeispiel 2: Inbetriebnahme einer Gasphasenphosgenierungsanlage (100) unter gleichzeitiger Einspeisung von Amin (2) und Phosgen (1)

Eine Gasphasenphosgenierungsanlage (**100**) wird, wie oben beschrieben, betrieben. Nach einer Betriebsunterbrechung wird die Anlage wie folgt wieder in Betrieb genommen: Der Aminverdampfer (**2000**) und die Leitung (**2100**) einschließlich der Amindüse werden mit einem Stickstoffgasstrom (**30**) beaufschlagt, wobei eine Temperatur von 380 °C eingestellt wird. Der Phosgenierungsreaktor (**3000**) ist Edukt- und Produkt-frei und wird mit heißem Stickstoff (**30**) inertisiert. Die Aminverdampfung im Aminverdampfer (**2000**) wird gestartet, TDA wird bei 300 °C verdampft, in einem weiteren Wärmeaustauscher auf 410 °C erhitzt und als gasförmiges TDA (**20**) bei einem absoluten Druck von 1.683 mbar durch die Amindüse in den Phosgenierungsreaktor (**3000**) eingedüst. Die Menge an TDA (**2**), die während einer geplanten Anfahrzeit von 45 Minuten in den Phosgenierungsreaktor (**3000**) eingeleitet wird, soll stufenlos von 0 t/h auf 12 t/h erhöht werden. Gleichzeitig mit dem Durchtritt des TDA-Gasstroms (**20**) durch die Amindüse in den Phosgenierungsreaktor (**3000**) wird die Phosgenzufuhr geöffnet und der Phosgengasstrom (**10**) mit einem Massenfluss von 61 t/h, einer Temperatur von 320 °C und einem absoluten Druck von 1.691 mbar am Reaktoreintritt in den Phosgenierungsreaktor (**3000**) eingedüst. Die Anlage kann nach 45 Minuten Anfahrzeit im normalen Betriebsmodus betrieben werden. Nach 5 Tagen muss die Anlage abgestellt werden, weil der Differenzdruck zwischen Eintritt der Edukte TDA-Gasstrom (**20**) und Phosgengasstrom (**10**) in den Phosgenierungsreaktor (**3000**) und Brüdengasaustritt des Sumpfes (**4100**) auf 793 mbar, statt 10 mbar bei Normalbetrieb, angestiegen ist, und die benötigte Verdampfungsenergie zum Verdampfen von Phosgen (**1**) und TDA (**2**) kaum noch aufgebracht werden kann. Nach Abstellung und Öffnen der Anlage werden am Mund der Amindüse, entlang der Oberfläche des Reaktorraumes und auf den Oberflächen der Quenchen Polyharnstoff-haltige massive Ablagerungen gefunden.

### Vergleichsbeispiel 3: Inbetriebnahme einer Gasphasenphosgenierungsanlage (100) unter Einspeisung von Phosgen (1) vor Amin (2), jedoch ohne Inertisierung der Aminzuführungseinrichtungen mit einem Inertgasstrom (30)

Es wird ein Phosgenkreislauf aufgebaut, indem Rezyklat-Phosgen (**1"**) aus der Aufarbeitung (**5100**) der Brüden (**50**) der Reaktionsabbruchzone (**4000**) mit einer Temperatur von 320 °C über den Phosgenierungsreaktor (**3000**), die Reaktionsabbruchzone (**4000**) zurück zur Aufarbeitung gefahren wird. In der Reaktionsabbruchzone (**4000**) ist währenddessen nur die in Strömungsrichtung des Reaktionsgemisches (**40**) zweite Quenche in Betrieb, wodurch der Phosgenstrom abgekühlt wird. In diesem Phosgenkreislauf werden 61 t/h Phosgen umgewälzt (Schritt (I)). Der Aminverdampfer (**2000**) und die Leitung **2100** einschließlich der Amindüse werden währenddessen nicht mit einem Stickstoffgasstrom (**30**) gespült. Sobald der Phosgenierungsreaktor (**3000**) auf 320 °C temperiert ist, wird die Aminverdampfung gestartet - ohne dass zuvor die Leitung (**2100**) einschließlich der Amindüse mit einem Stickstoffgasstrom (**30**) gespült wird - indem auf 220 °C vorgeheiztes, flüssiges TDA (**2**) gemeinsam mit Stickstoff in den Aminverdampfer (**2000**) gefahren wird, dort mit Hilfe eines Wärmeaustauschers bei 300 °C verdampft und anschließend mit einem weiteren Wärmeaustauscher auf 410 °C erhitzt wird. Der so erhaltene TDA-Strom (**20**) wird bei einem absoluten Druck von 1.654 mbar durch die Amindüse in den Phosgenierungsreaktor (**3000**) eingedüst (Schritt (III)). Die Menge an TDA (**2**), die während der Inbetriebnahme der Gasphasenphosgenierungsanlage (d. h. bis der Amin-Massenfluss M'_{Soll}(**2**) erreicht ist, was nach 45 Minuten der Fall ist) in den Phosgenierungsreaktor (**3000**) eingeleitet wird, wird stufenlos von 0 t/h auf 12 t/h erhöht, wobei der Betriebsdruck im Phosgenierungsreaktor (**3000**) am Ende der Inbetriebnahme 1.641 mbar (absolut) beträgt. Die in Strömungsrichtung des Reaktionsgemisches (**40**) erste Quenche wird in Betrieb genommen kurz bevor erstmalig TDA-Gasstrom (**20**) bei der Inbetriebnahme die Amindüse in Richtung des Phosgenierungsreaktors (**3000**) verlässt. Das nach dem Start der Anlage verbrauchte Phosgen wird durch eine Mischung aus frischem Phosgen (**1'**) und in der Aufarbeitung wiedergewonnenem Phosgen (**1"**) ersetzt. Nach 60 Minuten verlassen 15,6 t/h an TDI (**4**) die letzte Destillationskolonne der Aufarbeitungsstufe.

Nach dem Anfahren steigt der Differenzdruck zwischen Aminverdampfung (**2000**) und Phosgenierungsreaktor (**3000**) immer weiter an. Nach 3 Stunden muss der Phosgenierungsreaktor (**3000**) abgestellt werden, weil der Betriebsdruck an der Amindüse auf 2,5 bar (absolut) gestiegen ist. Nach Abstellung und Öffnen der Anlage werden an der Austrittsöffnung der Amindüse und in der zur Amindüse führenden Rohrleitung verkohlte Rückstände gefunden. Dies ist auf Rückströmen von Phosgen in die Einrichtung **2100** während der Anfahrphase zurückzuführen, wobei TDI-Ablagerungen entstehen, welche die Austrittsöffnung der Amindüse verstopfen.

### Beispiel 4 (erfindungsgemäß)

Es wird verfahren wie in Beispiel 3 beschrieben, mit der Ausnahme, dass, sobald der Phosgenierungsreaktor (**3000**) auf 320 °C temperiert ist, der Aminverdampfer (**2000**) und die Leitung (**2100**) einschließlich der Amindüse mit heißem Stickstoff (**30**) gespült werden, wobei eine Temperatur von 380 °C eingestellt wird (Schritt (II)). Nach 60 Minuten verlassen 15,6 t/h an TDI (**4**) die letzte Destillationskolonne der Aufarbeitung (**5200**).

Bei dieser Vorgehensweise werden die Verblockung der Amindüse und die Entstehung von Ablagerungen im Phosgenierungsreaktor (**3000**) während der Anfahrphase verhindert, sodass die Anlage (**100**) über einen langen Zeitraum von bis zu über einem Jahr gefahren werden kann. Die Entstehung von unerwünschten Nebenprodukten wie Polyharnstoffen etc. ist signifikant reduziert, und ein späteres Verschneiden der Anfahrware mit TDI höherer Reinheit kann unterbleiben.

## Patentansprüche

1. Verfahren zum Betreiben einer Gasphasenphosgenierungsanlage (**100**) zur Umsetzung eines Amins (**2**) mit Phosgen (**1**) zum korrespondierenden Isocyanat (**4**), wobei die Gasphasenphosgenierungsanlage (**100**) mindestens
(i) eine Vorrichtung **1000** zur Bereitstellung eines gasformigen Phosgenstroms (**10**),
(ii) eine Vorrichtung **2000** zur Bereitstellung eines gasformigen Aminstroms (**20**),
(iii) eine Mischzone (**3100**) zur Vermischung der Ströme (**10**) und (**20**), wobei die Mischzone durch Einrichtungen (**1100, 2100**) jeweils mit der Vorrichtung **1000** und der Vorrichtung **2000** verbunden ist,
(iv) eine strömungstechnisch nach der Mischzone (**3100**) angeordnete Reaktionszone (**3200**) zur weiteren Umsetzung der zuvor vermischten Ströme (**10**) und (**20**),
(v) eine strömungstechnisch nach der Reaktionszone (**3200**) angeordnete Reaktionsabbruchzone (**4000**) zur Beendigung der Umsetzung,
und optional
(vi) einen Aufarbeitungsteil (**5000**) umfassend Einrichtungen zur Rückgewinnung und Rückführung nicht umgesetzten Phosgens (**1"**) (**5100**) und Einrichtungen zur Gewinnung des hergestellten Isocyanats in Reinform **(5200),**
umfasst,
**dadurch gekennzeichnet, dass**
zur Inbetriebnahme der Gasphasenphosgenierungsanlage (**100**) die folgenden Schritte durchlaufen werden:
(I) Bereitstellung eines gasförmigen Phosgenstroms (**10**) einer Temperatur T₁₀ von 200 °C bis 600 °C mit einem absoluten Druck p₁₀ von 100 mbar bis 3.000 mbar, in der Vorrichtung **1000** und kontinuierliches Einbringen dieses gasförmigen Phosgentroms (**10**) durch die Einrichtung **1100** in die Mischzone (**3100**), die Reaktionszone (**3200**) und die Reaktionsabbruchzone (**4000**), wobei der Druck p₁₀ größer ist als der Druck p₃₁₀₀ in der Mischzone (**3100**);
(II) gleichzeitig mit oder nach Schritt (I),
(a) Einleiten eines, bevorzugt bei Raumtemperatur und Normaldruck flüssig vorliegenden, Inertstoffs (**3**) einer Temperatur T₃ < 200 °C in die Vorrichtung **2000,** Erhitzen des Inertstoffs (**3**) in der Vorrichtung **2000** unter Erhalt eines Inertgasstroms (**30**), welcher durch die Einrichtung **2100,** die Mischzone (**3100**), die Reaktionszone (**3200**) und die Reaktionsabbruchzone (**4000**) geleitet wird
oder
(b) Einleiten eines Inertgasstroms (**30**)
(b.1) in die Einrichtung **2100** und von dort durch die Mischzone (**3100**), die Reaktionszone (**3200**) und die Reaktionsabbruchzone (**4000**)
oder bevorzugt
(b.2) in die Vorrichtung **2000** und von dort durch die Einrichtung **2100,** die Mischzone (**3100**), die Reaktionszone (**3200**) und die Reaktionsabbruchzone **(4000)**
wobei in Variante (a) und in Variante (b) der Inertgasstrom (**30**) eine Temperatur von 200 °C bis 600 °C und einen absoluten Druck p₃₀ von 100 mbar bis 3.000 mbar aufweist,
wobei der Druck p₃₀ in beiden Fällen größer ist als der Druck p₃₁₀₀ in der Mischzone (**3100**);
(III) nach Schritt (II), Bereitstellung eines gasförmigen Aminstroms (**20**) einer Temperatur T₂₀ von 200 °C bis 600 °C mit einem absoluten Druck p₂₀ von 100 mbar bis 3.000 mbar in der Vorrichtung **2000** und kontinuierliches Einbringen dieses gasförmigen Aminstroms (**20**) durch die Einrichtung **2100** in die Mischzone (**3100**), wobei der Druck p₂₀ größer ist als der Druck p₃₁₀₀ in der Mischzone (**3100**), und wobei die Zusammensetzung und der Massenfluss des Stroms (**20**) so auf die Zusammensetzung und den Massenfluss des Stroms **10** abgestimmt werden, dass in der Mischzone (**3100**) Phosgen (**1**) in einem stöchiometrischen Überschuss in Bezug auf die primären Aminogruppen des Amins (**2**) vorliegt.

2. Verfahren nach Anspruch 1, bei dem die Gasphasenphosgenierungsanlage (**100**) den Aufarbeitungsteil (**5000**) umfasst und bei dem im Regelbetrieb der Gasphasenphosgenierungsanlage (**100**) das Phosgen (**1**) im Phosgengasstrom (**10**) eine Mischung aus Frisch-Phosgen (**1'**) und in den Einrichtungen **5100** zurückgewonnenem Rezyklat-Phosgen (**1"**) ist.

3. Verfahren nach Anspruch 2, bei dem das Phosgen im Phosgengasstrom (**10**) aus Schritt (I) zumindest teilweise aus Rezyklat-Phosgen (**1"**) besteht.

4. Verfahren nach einem der Ansprüche 1 bis 3, bei dem die Zusammensetzungen und Massenflüsse der Ströme **10** und **20** so aufeinander abgestimmt werden, dass in Schritt (III) in der Mischzone (**3100**) Phosgen (**1**) in einem stöchiometrischen Überschuss in Bezug auf die primären Aminogruppen des Amins (**2**) von mindestens 150 % der Theorie vorliegt.

5. Verfahren nach einem der Ansprüche 1 oder 2, bei dem die Drücke p₁₀ und p₂₀ gleich sind.

6. Verfahren nach einem der Anspräche 1 bis 3, bei dem der Massenfluss des Stroms **20** stufenlos oder in Stufen, bevorzugt stufenlos, auf einen angestrebten Soll-Wert M'_{Soll}(**20**) gesteigert wird.

7. Verfahren nach Anspruch 6, bei dem der Massenfluss des Stroms **20** so gesteigert wird, dass innerhalb von maximal 12 Stunden mindestens 80 % des angestrebten Soll-Werts MM'_{Soll}(**20**) erreicht werden.

8. Verfahren nach einem der Ansprüche 1 bis 5, bei dem der absolute Druck p₃₁₀₀ auf einen Wert von 80 mbar bis 2.500 mbar eingestellt wird.

9. Verfahren nach einem der Ansprüche 1 bis 6, bei dem die Einrichtung **1100** eine Düse umfasst.

10. Verfahren nach einem der Ansprüche 1 bis 7, bei dem die Einrichtung **2100** eine Düse umfasst.

11. Verfahren nach einem der Ansprüche 1 bis 6, bei dem die Einrichtungen **1100** und **2100** eine gemeinsame Düsenvorrichtung umfassen.

12. Verfahren nach einem der Ansprüche 1 bis 11, bei dem der Inertgasstrom (**30**) während der Durchführung von Schritt (III) aufrechterhalten wird.

13. Verfahren nach einem der Ansprüche 1 bis 12, bei dem die Reaktionsabbruchzone (**4000**) durch Kontaktieren des aus der Reaktionszone (**3200**) austretenden gasförmigen Verfahrensprodukts mit einem inerten Lösungsmittel unterhalb der Siedetemperatur des Isocyanats (**4**) und oberhalb der Zersetzungstemperatur des zum Amin (**2**) korrespondieren Carbamoylchlorids betrieben wird.

14. Verfahren nach Anspruch 13, bei dem die Reaktionsabbruchzone spätestens dann in Betrieb genommen wird, wenn in Schritt (III) erstmalig Strom (**20**) in die Mischzone (**3100**) eintritt.

15. Verfahren nach einem der Ansprüche 1 bis 14, bei dem das Amin (**2**) ausgewählt ist aus der Gruppe bestehend aus Isophorondiamin, Hexamethylendiamin, Bis(p-aminocyclohexyl)methan, Toluylendiamin und Diphenylmethandiamin.

## Claims

1. Method of operating a gas phase phosgenation plant (**100**) for reacting an amine (**2**) with phosgene (**1**) to give the corresponding isocyanate (**4**), said gas phase phosgenation plant (**100**) comprising at least
(i) an apparatus **1000** for providing a gaseous phosgene stream (**10**),
(ii) an apparatus **2000** for providing a gaseous amine stream (**20**),
(iii) a mixing zone (**3100**) for mixing the streams (**10**) and (**20**), the mixing zone being connected by each of devices (**1100, 2100**) to the apparatus **1000** and the apparatus **2000,**
(iv) a reaction zone (**3200**) arranged downstream of the mixing zone (**3100**) for further conversion of the previously mixed streams (**10**) and (**20**),
(v) a reaction stopping zone (**4000**) arranged downstream of the reaction zone (**3200**) to end the reaction,
and optionally
(vi) a workup section (**5000**) comprising devices for recovery and recycling of unconverted phosgene (**1"**) (**5100**) and devices for obtaining the isocyanate prepared in pure form (**5200**),
**characterized in that**
the gas phase phosgenation plant (**100**) is started up by running the following steps:
(I) providing a gaseous phosgene stream (**10**) at a temperature T₁₀ of 200°C to 600°C with an absolute pressure p₁₀ of 100 mbar to 3000 mbar in the apparatus **1000** and continuously introducing this gaseous phosgene stream (**10**) through the device **1100** into the mixing zone (**3100**), the reaction zone (**3200**) and the reaction stopping zone (**4000**), the pressure p₁₀ being greater than the pressure p₃₁₀₀ in the mixing zone (**3100**);
(II) simultaneously with or after step (I),
(a) introducing an inert substance (**3**), preferably one which is liquid at room temperature and standard pressure, at a temperature T₃ < 200°C into the apparatus **2000,** heating the inert substance (**3**) in the apparatus **2000** to obtain an inert gas stream (**30**) which is passed through the device **2100,** the mixing zone (**3100**), the reaction zone (**3200**) and the reaction stopping zone (**4000**) or
(b) introducing an inert gas stream (**30**)
(b.1) into the device **2100** and thence through the mixing zone (**3100**), the reaction zone (**3200**) and the reaction stopping zone (**4000**) or preferably
(b.2)into the apparatus **2000** and thence through the device **2100,** the mixing zone (**3100**), the reaction zone (**3200**) and the reaction stopping zone (**4000**),
where the inert gas stream (**30**) in variant (a) and in variant (b) has a temperature of 200°C to 600°C and an absolute pressure p₃₀ of 100 mbar to 3000 mbar,
where the pressure p₃₀ in both cases is greater than the pressure p₃₁₀₀ in the mixing zone (**3100**);
(III) after step (II), providing a gaseous amine stream (**20**) at a temperature T₂₀ of 200°C to 600°C with an absolute pressure p₂₀ of 100 mbar to 3000 mbar in the apparatus **2000** and continuously introducing this gaseous amine stream (**20**) through the device **2100** into the mixing zone (**3100**), where the pressure p₂₀ is greater than the pressure p₃₁₀₀ in the mixing zone (**3100**), and where the composition and the mass flow rate of the stream (**20**) are matched to the composition and the mass flow rate of stream **10** such that, in the mixing zone (**3100**), phosgene (**1**) is present in a stoichiometric excess in relation to the primary amino groups of the amine (**2**).

2. Method according to Claim 1, in which the gas phase phosgenation plant (**100**) includes the workup section (**5000**) and in which, in the regular operation of the gas phase phosgenation plant (**100**), the phosgene (**1**) in the phosgene gas stream (**10**) is a mixture of fresh phosgene (**1'**) and recycled phosgene (**1"**) recovered in the devices **5100.**

3. Method according to Claim 2, in which the phosgene in the phosgene gas stream (**10**) from step (I) consists at least partly of recycled phosgene (**1"**).

4. Method according to any of Claims 1 to 3, in which the compositions and mass flow rates of streams **10** and **20** are matched to one another such that, in step (III), in the mixing zone (**3100**), phosgene (**1**) is present in a stoichiometric excess in relation to the primary amino groups of the amine (**2**) of at least 150% of theory.

5. Method according to either of Claims 1 and 2, in which the pressures p₁₀ and p₂₀ are the same.

6. Method according to any of Claims 1 to 3, in which the mass flow rate of stream **20** is increased continuously or in stages, preferably continuously, to a desired target value M' target (**20**).

7. Method according to Claim 6, in which the mass flow rate of stream **20** is increased such that at least 80% of the desired target value M'target (**20**) is attained within not more than 12 hours.

8. Method according to any of Claims 1 to 5, in which the absolute pressure p₃₁₀₀ is adjusted to a value of 80 mbar to 2500 mbar.

9. Method according to any of Claims 1 to 6, in which the device **1100** comprises a nozzle.

10. Method according to any of Claims 1 to 7, in which the device **2100** comprises a nozzle.

11. Method according to any of Claims 1 to 6, in which the devices **1100** and **2100** comprise a common nozzle apparatus.

12. Method according to any of Claims 1 to 11, in which the inert gas stream (**30**) is maintained during the performance of step (III).

13. Method according to any of Claims 1 to 12, in which the reaction stopping zone (**4000**) is operated below the boiling temperature of the isocyanate (**4**) and above the breakdown temperature of the carbamoyl chloride corresponding to the amine (**2**) by contacting the gaseous process product exiting from the reaction zone (**3200**) with an inert solvent.

14. Method according to Claim 13, in which the reaction stopping zone is put into operation no later than when stream (**20**) enters the mixing zone (**3100**) for the first time in step (III).

15. Method according to any of Claims 1 to 14, in which the amine (**2**) is selected from the group consisting of isophoronediamine, hexamethylenediamine, bis(p-aminocyclohexyl)methane, tolylenediamine and diphenylmethanediamine.

## Revendications

1. Procédé d'exploitation d'une unité de phosgénation en phase gazeuse (100) pour la mise en réaction d'une amine (2) avec du phosgène (1) pour former l'isocyanate correspondant (4), l'unité de phosgénation en phase gazeuse (100) comprenant au moins :
(i) un dispositif 1000 pour la préparation d'un courant de phosgène gazeux (10),
(ii) un dispositif 2000 pour la préparation d'un courant d'aminé gazeux (20),
(iii) une zone de mélange (3100) pour le mélange des courants (10) et (20), la zone de mélange étant reliée par des appareils (1100, 2100) respectivement avec le dispositif 1000 et le dispositif 2000,
(iv) une zone de réaction (3200) agencée en termes d'écoulement après la zone de mélange (3100) pour la mise en réaction ultérieure des courants (10) et (20) mélangés auparavant,
(v) une zone d'arrêt de la réaction (4000) agencée en termes d'écoulement après la zone de réaction (3200) pour la fin de la réaction,
et éventuellement
(vi) une partie de traitement (5000) comprenant des appareils pour la récupération et le recyclage du phosgène non réagi (1") (5100) et des appareils pour l'obtention de l'isocyanate fabriqué sous forme pure (5200),
**caractérisé en ce que**
les étapes suivantes sont réalisées pour la mise en service de l'unité de phosgénation en phase gazeuse (100) :
(I) la préparation d'un courant de phosgène gazeux (10) d'une température T₁₀ de 200 °C à 600 °C avec une pression absolue p₁₀ de 100 mbar à 3 000 mbar dans le dispositif 1 000, et l'introduction continue de ce courant de phosgène gazeux (10) par l'appareil 1100 dans la zone de mélange (3100), la zone de réaction (3200) et la zone d'arrêt de la réaction (4000), la pression p₁₀ étant supérieure à la pression p₃₁₀₀ dans la zone de mélange (3100) ;
(II) simultanément à ou après l'étape (I),
(a) l'introduction d'une substance inerte (3), de préférence se présentant sous forme liquide à température ambiante et pression normale, d'une température T₃ < 200 °C dans le dispositif 2000, le chauffage de la substance inerte (3) dans le dispositif 2000 avec obtention d'un courant de gaz inerte (30), qui est conduit dans l'appareil 2100, la zone de mélange (3100), la zone de réaction (3200) et la zone d'arrêt de la réaction (4000),
ou
(b) l'introduction d'un courant de gaz inerte (30)
(b.1) dans l'appareil 2100 et depuis celui-ci dans la zone de mélange (3100), la zone de réaction (3200) et la zone d'arrêt de la réaction (4000),
ou de préférence
(b.2) dans le dispositif 2000 et depuis celui-ci dans l'appareil 2100, la zone de mélange (3100), la zone de réaction (3200) et la zone d'arrêt de la réaction (4000),
selon la variante (a) et selon la variante (b), le courant de gaz inerte (30) présentant une température de 200 °C à 600 °C et une pression absolue p₃₀ de 100 mbar à 3 000 mbar,
la pression p₃₀ dans les deux cas étant supérieure à la pression p₃₁₀₀ dans la zone de mélange (3100) ;
(III) après l'étape (II), la préparation d'un courant d'amine gazeux (20) d'une température T₂₀ de 200 °C à 600 °C avec une pression absolue p₂₀ de 100 mbar à 3 000 mbar dans le dispositif 2000, et l'introduction continue de ce courant d'amine gazeux (20) par l'appareil 2100 dans la zone de mélange (3100), la pression p₂₀ étant supérieure à la pression p₃₁₀₀ dans la zone de mélange (3100), et la composition et le débit massique du courant (20) étant accordés à la composition et au débit massique du courant 10 de sorte que le phosgène (1) soit présent dans la zone de mélange (3100) en un excès stoechiométrique par rapport aux groupes amino primaires de l'amine (2).

2. Procédé selon la revendication 1, selon lequel l'unité de phosgénation en phase gazeuse (100) comprend la partie de traitement (5000) et selon lequel, en mode normal de l'unité de phosgénation en phase gazeuse (100), le phosgène (1) dans le courant gazeux de phosgène (10) est un mélange de phosgène frais (1') et de phosgène recyclé (1") récupéré dans les appareils 5100.

3. Procédé selon la revendication 2, selon lequel le phosgène dans le courant gazeux de phosgène (10) de l'étape (I) est au moins partiellement constitué de phosgène recyclé (1").

4. Procédé selon l'une quelconque des revendications 1 à 3, selon lequel les compositions et les débits massiques des courants 10 et 20 sont accordés les uns aux autres de sorte qu'à l'étape (III), le phosgène (1) soit présent dans la zone de mélange (3100) en un excès stoechiométrique par rapport aux groupes amino primaires de l'amine (2) d'au moins 150 % de la théorie.

5. Procédé selon l'une quelconque des revendications 1 ou 2, selon lequel les pressions p₁₀ et p₂₀ sont identiques.

6. Procédé selon l'une quelconque des revendications 1 à 3, selon lequel le débit massique du courant 20 est augmenté de manière continue ou par paliers, de préférence de manière continue, à une valeur de consigne visée M'_{Soll} (20).

7. Procédé selon la revendication 6, selon lequel le débit massique du courant 20 est augmenté de sorte qu'au moins 80 % de la valeur de consigne visée M'_{Soll} (20) soit atteinte en au plus 12 heures.

8. Procédé selon l'une quelconque des revendications 1 à 5, selon lequel la pression absolue p₃₁₀₀ est ajustée à une valeur de 80 mbar à 2 500 mbar.

9. Procédé selon l'une quelconque des revendications 1 à 6, selon lequel l'appareil 1100 comprend une buse.

10. Procédé selon l'une quelconque des revendications 1 à 7, selon lequel l'appareil 2100 comprend une buse.

11. Procédé selon l'une quelconque des revendications 1 à 6, selon lequel les appareils 1100 et 2100 comprennent un dispositif à buse commun.

12. Procédé selon l'une quelconque des revendications 1 à 11, selon lequel le courant de gaz inerte (30) est maintenu pendant la réalisation de l'étape (III).

13. Procédé selon l'une quelconque des revendications 1 à 12, selon lequel la zone d'arrêt de la réaction (4000) est exploitée par mise en contact du produit de procédé gazeux sortant de la zone de réaction (3200) avec un solvant inerte en dessous de la température d'ébullition de l'isocyanate (4) et au-dessus de la température de décomposition du chlorure de carbamoyle correspondant à l'amine (2).

14. Procédé selon la revendication 13, selon lequel la zone d'arrêt de la réaction est mise en service au plus tard lorsque le courant (20) entre pour la première fois dans la zone de mélange (3100) à l'étape (III).

15. Procédé selon l'une quelconque des revendications 1 à 14, selon lequel l'amine (2) est choisie dans le groupe constitué par l'isophorone-diamine, l'hexaméthylène-diamine, le bis(p-aminocyclohexyl)méthane, la toluylène-diamine et la diphénylméthane-diamine.
